# EUROPÄISCHE PATENTANMELDUNG

(11) **EP 0 979 997 A1**
(43) Veröffentlichungstag der Anmeldung: **16.02.2000**
(21) Anmeldenummer: 99109096.0
(22) Anmeldetag: 07.05.1999
(51) Int. Cl.: G01N 33/00, G01N 27/12, B09B 1/00

(54) **Verfahren und Vorrichtung zum Identifizieren einer von einem Lebewesen abgesonderten Flüssigkeit, insbesondere von Fruchtwasser**

(30) Priorität: 12.08.1998 DE 19836591
(71) Anmelder: SIEMENS AKTIENGESELLSCHAFT, 80333 München (DE)
(72) Erfinder: Abraham-Fuchs, Klaus, 91058 Erlangen (DE); Schmidt, Kai-Uwe Dr., 91052 Erlangen (DE); Tork, Joachim, 91052 Erlangen (DE); Wildt, Ludwig Prof.Dr., 91074 Herzogenaurauch (DE); Binder, Helge Dr., 91074 Herzogenaurauch (DE)

(57) **Zusammenfassung**

Es ist ein Verfahren zum Identifizieren einer von einem Lebewesen abgesonderten Flüssigkeit, insbesondere von Fruchtwasser, beschrieben, wobei die Flüssigkeit über deren charakteristische flüchtige Bestandteile identifiziert wird.

## Beschreibung

Die Erfindung betrifft ein Verfahren zum Identifizieren einer von einem Lebewesen abgesonderten Flüssigkeit, insbesondere von Fruchtwasser, sowie eine Vorrichtung zur Durchführung des Verfahrens.

Bei einer Schwangerschaft wird unter vorzeitigem Blasensprung der Abgang von Fruchtwasser mit Eröffnung der Fruchtblase bei wehenlosem Uterus verstanden. Die klinische Bedeutung des vorzeitigen Blasensprungs liegt in der Tatsache begründet, daß eine freie Verbindung zwischen Scheide und Außenwelt einerseits und intrauteriner Fruchthülle gegeben ist, wodurch auch der Zutritt von Keimen mit nachfolgender intrauteriner Infektion ermöglicht wird. Eine intrauterine Infektion kann sowohl für das Kind als auch für die Mutter lebensbedrohend werden, es kann darüber hinaus zu einer dauernden Sterilität führen.

Während der manifeste Blasensprung mit schwallartigem Abgang von Fruchtwasser keine diagnostischen Probleme aufwirft, ist der sogenannte hohe Blasensprung oft nur sehr schwer nachzuweisen. Die Patientinnen geben den Abgang von Flüssigkeit an, wobei nicht klar ist, ob es sich um Fruchtwasser, Urin oder Scheidensekret handelt.

Der Nachweis des vorzeitigen Blasensprungs ist von erheblicher klinisch geburtshilflicher Bedeutung. Die Risiken, die mit einem nicht erkannten Blasensprung verbunden sind, sind schwerwiegend und unter Umständen lebensbedrohend. Sie können besonders in der Frühschwangerschaft eine Frühgeburt mit all ihren Begleiterscheinungen zur Folge haben. Der sichere Ausschluß eines vorzeitigen Blasensprungs ist ebenfalls von großer Bedeutung, da eine entsprechende Symptomatik (Abgang von Flüssigkeit) häufig beobachtet wird und es dann oft unnötig zur stationären Aufnahme in einem Krankenhaus mit entsprechenden Unannehmlichkeiten und Kosten kommt.

Zum Nachweis von Fruchtwasser sind verschiedene Verfahren eingesetzt worden. Diese beruhen im wesentlichen auf dem Nachweis von Substanzen, die im Fruchtwasser in hoher Konzentration enthalten sind, im Vaginalsekret jedoch nicht vorkommen. Dazu gehören z. B. Prolactin, humanes Choriongonadotropin (HCG), Alpha-Feto-Protein (AFP) und andere schwangerschaftsspezifische Proteine. Die Problematik des Nachweises dieser Substanzen ist einerseits dadurch begründet, daß diese im sauren Vaginalsekret sehr schnell abgebaut werden und dann nicht mehr nachweisbar sind, zum anderen ist der Nachweis dieser Proteine methodisch oft nicht einfach durchzuführen.

Eine weitere Diagnosemöglichkeit besteht darin, Ultraschalluntersuchungen durchzuführen. Dabei wird versucht, durch Pressen und Schütteln des Unterleibs einen Abfluß von Fruchtwasser außen entlang der Fruchtblase zu provozieren. Dieser soll im Fall eines positiven Befundes im Ultraschallbild erkennbar sein.

Eine andere Möglichkeit zur Diagnose besteht in der Ausnutzung der Tatsache, daß das Fruchtwasser einen alkalischen pH-Wert besitzt. Dies wird durch einen Lackmustest der Vaginalflüssigkeit überprüft.

Aus der DE 37 06 372 C2 ist ein Verfahren und eine Vorrichtung zur Geburtsmeldung bei Tieren, insbesondere bei Hunden, bekannt. Das Verfahren sieht vor, daß die durch das bei der Geburt freiwerdende Fruchtwasser schlagartig sich erhöhende Luftfeuchtigkeit ermittelt und unmittelbar danach ein akustisches und/oder optisches Signal ausgelöst wird. Eine Vorrichtung zur Durchführung des Verfahrens sieht vor, daß innerhalb einer Hundehütte oder dergleichen, vorzugsweise im Deckenbereich, ein Feuchtefühler angeordnet ist, der mit einem plötzliche Luftfeuchtigkeitsveränderungen registrierenden, elektrisch betätigbaren Auswertegerät verbunden ist. Dies ist dabei impulsgebend mit einem außerhalb der Hundehütte vorgesehenen Signalgeber verbunden. Dieses Verfahren und diese Vorrichtung sind jedoch nicht geeignet, eine von einem Lebewesen abgesonderte Flüssigkeit zu charakterisieren. Da Lebewesen zu 70-80% aus Wasser bestehen, sind auch Proben von Lebewesen immer feucht. Das Vorhandensein von Feuchtigkeit ist nicht charakteristisch für eine bestimmte abgesonderte Flüssigkeit, insbesondere kann nicht unterschieden werden, ob es sich um Fruchtwasser, Urin oder Scheidensekret handelt.

Unter der Bezeichnung elektronische Nase sind Sensorsysteme beschrieben, die von ihrer Funktionsweise dem menschlichen Geruchssinn nahe sind. Sie erfassen im Gegensatz zur Gaschromatographie oder Massenspektroskopie nicht einzelne Komponenten eines Gases, sondern Summenparameter. Es wird ein sogenannter Fingerabdruck des Geruchs aufgezeichnet. Die Ergebnisse werden nach Art der Auswertung unterschiedlich dargestellt.

So ist in dem Artikel von M.A. Craven, J.W. Gardner, P.N. Bartlett: "Electronic noses - development and future prospects", erschienen in trends in analytical chemistry, Vol. 355 (1996), pp. 486-493, angegeben, daß nun kommerzielle Geräte, die elektronische Nasen genannt werden, in einer Vielzahl von Industriezweigen, wie Nahrungsmittel, Wasser, Brauerei, eingesetzt werden. Als mögliches zukünftiges Anwendungsgebiet von elektronischen Nasen ist dort auch die medizinische Diagnostik genannt.

Der Erfindung liegt nun die Aufgabe zugrunde, ein Verfahren und eine Vorrichtung anzugeben, womit auf einfache Art eine von einem Lebewesen abgesonderte Flüssigkeit, insbesondere Fruchtwasser, identifiziert werden kann.

Die Aufgabe erstgenannte wird dadurch gelöst, daß die Flüssigkeit über deren charakteristische flüchtige Bestandteile identifiziert wird. Dem liegt die Erfahrung zugrunde, daß derartige Flüssigkeiten unabhängig vom Individuum einen charakteristischen Geruch besitzen. Die Flüssigkeiten besitzen differenziert nach ihren Inhalten einen bestimmten Dampfdruck, so daß charakteristische Gase in der Umgebungsluft nachweisbar werden. Die Gasanalyse ist gegenüber einer Flüssigkeitsanalyse einfacher, weil die charakterisierenden Inhaltsstoffe in der komplexen aber uncharakteristischen Flüssigkeitsmatrix schwer nachweisbar sind und weil bei der Flüssigkeitsanalyse oft nach der Probennahme zusätzlich eine Probenaufbereitung erforderlich ist.

Prinzipiell kann durch den Einsatz spezifischer Gassensoren jede einzelne Gasart bzw. deren Bestandteile und Zusammensetzung gemessen werden. Viele spezifische Gassensoren sind nötig, um einen komplexen Duft, der aus vielen Bestandteilen oder Analyten besteht, auf diese Weise zu erkennen. Neben der schwierigen technischen Realisierung dieser Sensoren sind eine Vielzahl von Sensoren notwendig, die dann für sich zusammengenommen wieder nur zur Charakterisierung weniger Gerüche einsetzbar sind. Dazu kommt noch, daß im allgemeinen wissenschaftlich noch nicht exakt belegt ist, wieviel und welche chemischen Stoffe einen komplexen Geruch ergeben. Nichtsdestoweniger macht ein solcher Ansatz da Sinn, wo der Geruch durch einen bzw. wenige für diesen Geruch erkannte chemische Stoffe verursacht wird, wie dies z. B. beim diabetischen Koma, wo Aceton und Ketonverbindungen freigesetzt werden, der Fall ist.

Je nach Anwendungsfall kann bei zu geringer Analytkonzentration eine Anreicherung der Analyte nötig sein. Diese wird durch ein Adsorptionsröhrchen realisiert, durch das die Probe über ein Adsorbens geführt wird. Dabei werden die Analyte darin gefangen. Mit Hilfe einer nachfolgenden Thermodesorption können die Stoffe in einem entsprechend kleineren Volumen freigesetzt werden, wodurch die Konzentration erhöht wird. Die Sensitivität des Gesamtsystems kann so verbessert werden.

Demgegenüber werden gemäß einer besonders vorteilhaften Ausgestaltung die charakteristischen flüchtigen Bestandteile mehreren analytunspezifischen Sensoren zugeführt, die untereinander verschiedenes Sensorverhalten besitzen, und wobei von den charakteristischen flüchtigen Bestandteilen erzeugte Meßsignale der Sensoren einem Rechner zur Typisierung und Clusterung zugeführt werden. Eine Anordnung von analytunspezifischen Sensoren mit einer entsprechenden Auswertung wird auch als elektronische Nase bezeichnet. Setzt man mehrere solcher Sensoren ein, typisch 8 bis 32, die untereinander unterschiedliches Sensorverhalten aufweisen, erhält man je nach Geruch unterschiedliche Meßwerte oder Sensorantworten, sozusagen einen Fingerabdruck (finger print) des jeweiligen Analyten. Idealerweise decken die Sensoren ein weites Spektrum chemischer Eigenschaften der Gasanalyten ab, wie z. B. die Molekülgröße, das Redoxverhalten usw., damit keine redundante Information durch verschiedene Sensoren gemessen wird. Ein exakter Fingerabdruck des Geruchs ist aber nicht zu erwarten. Anzustreben ist vielmehr der Aufbau eines Pools oder eine Datenbank von Fingerabdrücken, unter dessen Verwendung, z. B. unter Einsatz mathematischer Methoden, eine Clusterung vorgenommen werden kann. Ein gemessenes Gasgemisch wird somit einem Cluster bzw. einem Geruchstyp zugeordnet. Dabei ist nicht ausgeschlossen, bei einem Geruch mit näher identifizierter Analytzusammensetzung die Sensoren zu spezifizieren. Insofern nähern sich beide Konzepte der Gasanalyse mit Hilfe von Gassensoren dann einander an.

Eine weitere vorteilhafte Ausgestaltung zeichnet sich dadurch aus, daß die charakteristischen flüchtigen Bestandteile mindestens einem Metalloxid-Sensor zugeführt werden. Das Sensorprinzip verwendet halbleitende Metalloxide, deren elektrischer Widerstand von der Analytzusammensetzung abhängt. Der Meßeffekt basiert auf der Änderung des Ladungstransfers mit Molekülen in der Gasphase, so daß charakteristische Änderungen der Leitfähigkeit bezüglich eines Analyts identifiziert werden können. Die Sensoren gibt es in einer Vielzahl von Ausführungen auch als spezifische Gassensoren.

Metalloxid-Sensorelemente sind jedoch nicht zum Erkennen aller möglichen Biomoleküle, insbesondere großer Moleküle, verwendbar. Derartige Sensorelemente müssen auf auf eine hohe Temperaturen aufgeheizt werden, zudem verändern sie die Biomoleküle während des Meßvorgangs. Abhilfe kann ein Katalysator schaffen, der die Biomoleküle aufsplittet und somit eine Untersuchung der charakteristischen Fragmente ermöglicht. Ebenfalls bedeutsam ist der hohe Energieverbrauch dieser Sensoren.

Bei einer weiteren vorteilhaften Ausgestaltung werden die charakteristischen flüchtigen Bestandteile mindestens einem Leitfähigkeitssensor zugeführt. Dieser Sensortyp benutzt leitfähige oder nicht-leitfähige Polymere. Sie arbeiten ähnlich wie die Metalloxid-Sensoren. Durch Verwendung unterschiedlicher Polymermaterialien, synthetischer Veränderung funktioneller Gruppen sowie durch Zusetzen unterschiedlicher Additiva kann eine große Zahl an sensitiven Varianten erzeugt werden. Grundsätzlich zeigen Polymer-Gassensoren eine gute Stabilität gegenüber Umwelteinflüssen, insbesondere gegen Temperatur. Sie reagieren im Gegensatz zu den Metalloxid-Sensoren auch nicht aktiv mit dem Analyt (Oxidation), so daß in der Rezeptorschicht keine Energiezufuhr nötig ist. Im Gegensatz zu den Metalloxid-Sensoren ist die Sensorantwort bei diesem Sensortyp von der Luftfeuchtigkeit abhängig. Auf nicht-polare Moleküle reagieren sie wenig sensitiv.

Bei einer weiteren vorteilhaften Ausgestaltung werden die charakteristischen flüchtigen Bestandteile mindestens einem Quartz-Micro-Balances-Sensor zugeführt. Quartz-Micro-Balances-Sensoren (QMB-Sensoren) bestehen im wesentlichen aus einem piezoelektrischen Quarzkristall. Als Meßeffekt wird dabei die Frequenzänderung des schwingenden Kristalls genutzt, die unter anderem durch auf der Oberfläche anlagernden Massen des Analyts verändert wird. Die Masseänderung durch Anlagerung der Analytmoleküle wird detektiert. Der typische Betriebsfrequenzbereich liegt zwischen 1 MHz und 30 MHz. Dieses Senorprinzip besitzt fast keine Temperaturabhängigkeit.

Bei einer weiteren vorteilhaften Ausgestaltung werden die charakteristischen flüchtigen Bestandteile mindestens einem Surface-Acoustic-Wave-Sensor zugeführt. Bei den Surface-Acoustic-Wave-Sensoren (SAW-sensoren) handelt es sich um ein Bauelement, auf dem die Ausbreitung von Schallwellen als selektive Eigenschaft für ein Analyt verwendet wird. Eine entsprechende Beschichtung macht das Sensorelement sensitiv für das jeweilige Analyt. Ausgewertet wird meist die Phasenverschiebung einer sich über das Element bewegenden Schallwelle, die von einem Reflektor zurückgeworfen wird. Die typische Oszillatorfrequenz liegt bei 430 MHz oder auch bei 1 GHz. Besonderes Augenmerk muß auf die Signalstabilität gerichtet werden. Einen großen Einfluß üben Temperaturschwankungen aus, die entweder durch Temperaturregelschaltungen oder durch ein zusätzliches Referenz-SAW-Bauteil vermindert werden können. Einen ähnlichen großen Einfluß übt die Luftfeuchtigkeit auf die Phasenverschiebungsmessung aus, der durch eine Absorption von Wassermolekülen durch den Quarz und der dadurch verursachten Kühlung hervorgerufen wird. Abhilfe kann hier eine Passivierungsschicht aus z. 8. SiNe erbringen, die unter anderem den Quarz abschirmt und ebenfallls als gute Grundlage für das Aufbringen der sensitiven Schicht angesehen werden kann.

Manchmal ist es nicht möglich, ein entsprechendes Gerät zur Identifizierung der flüchtigen Bestandteile unmittelbar am Anwendungsort, z. B. neben einem Patienten zu betreiben. Dann wird gemäß einer vorteilhaften Ausgestaltung eine Probe der Flüssigkeit mittels eines Probenträgers entnommen und einem mit den Sensoren verbundenen Probenbehälter zugeführt.

Eine kontinuierliche Identifizierung einer bestimmten Flüssigkeit kann dann erfolgen, wenn gemäß einer weiteren vorteilhaften Ausgestaltung die charakteristischen flüchtigen Bestandteile direkt vom Lebewesen abgesaugt werden.

Die zweitgenannte Aufgabe wird durch eine Vorrichtung gelöst, umfassend mehrere analytunspezifische Sensoren, mit den Sensoren verbundene Mittel zur Zuführung von charakteristischen flüchtigen Bestandteilen einer zu identifizierenden Flüssigkeit eines Lebewesens und einer mit den Sensoren verbundenen Auswerteeinheit zur Typisierung und Clusterung von den Sensoren abgegebener Meßsignale.

Zwei Ausführungsbeispiele der Erfindung werden im folgenden anhand von zwei Figuren erläutert. Es zeigen:
- FIG 1: ein erster Verfahren zum Identifizieren einer von einem Lebewesen abgesonderten Flüssigkeit, wobei die Flüssigkeit als Probe entnommen wird, und
- FIG 2: ein zweites Verfahren zum Identifizieren einer von einem Lebewesen abgesonderten Flüssigkeit mit einer direkten Probennahme der flüchtigen Bestandteile der zu analysierenden Flüssigkeit.

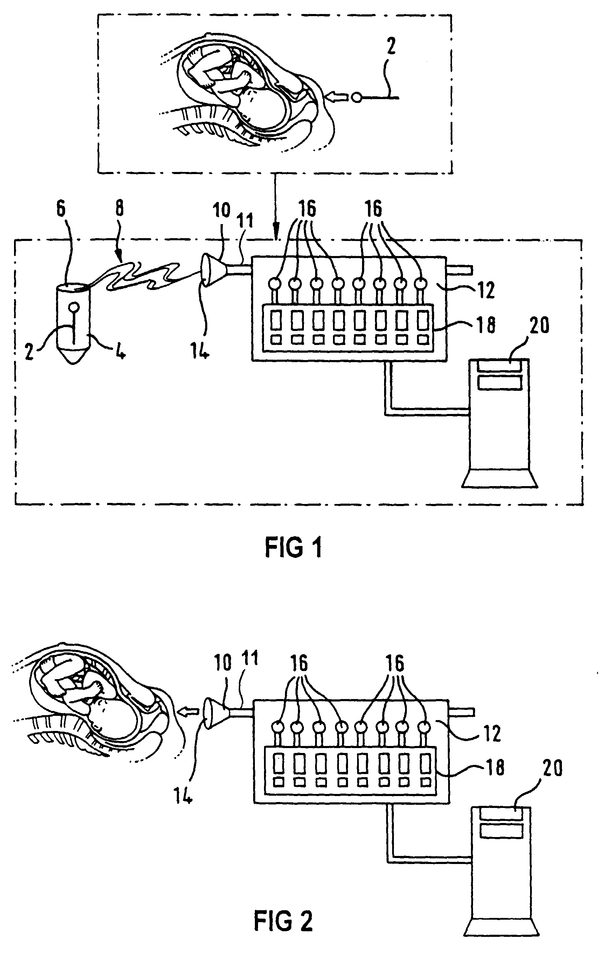

FIG 1 zeigt ein indirektes Verfahren zum Identifizieren einer von einem Lebewesen abgesonderten Flüssigkeit, hier Fruchtwasser, wobei die Flüssigkeit durch einen Probenabstrich (Vaginalsekret) mittels eines Probenträgers 2 in Form eines Wattestäbchens genommen wird. Der Probenträger 2 mit der zu identifizierenden Flüssigkeit wird in einem Probenbehälter 4 aufbewahrt und dann untersucht. Der Probenbehälter besitzt eine Öffnung 6, wo flüchtige Bestandteile 8 der Flüssigkeit austreten können. Die flüchtigen Bestandteile 8 werden über eine Probennahmeeinheit 10 einer Meßkammer 12 und ggf. einer Anreicherungseinheit 11 zugeführt. Die Probennahmeeinheit 10 umfaßt einen Saugrüssel 14 sowie - hier nicht dargestellt - eine Pumpe und ein Dosimeter oder Flowmeter. Die Anreicherungseinheit 11 besteht im wesentlichen aus einem Röhrchen, worin sich ein Adsorbens befindet. Mittels eines Heizelements werden die von dem Adsorbens zunächst festgehaltenen Moleküle wieder freigesetzt (Thermodesorption).

In der Meßkammer 12 sind eine Vielzahl von Sensoren 16, typisch 8 bis 32, angeordnet. Die Sensoren 16 besitzen unterschiedliches Sensorverhalten, was bewirkt, daß ein Analyt verschiedene Sensorantworten oder Meßsignale an den einzelnen Sensoren 16 erzeugt. Die Sensoren 16 umfassen Metalloxid-Sensoren und/oder Leitfähigkeitssensoren. Zur Verbesserung der Aussagequalität über die zu identifizierende Flüssigkeit und bei schwierigen Randbedingungen bietet sich an, weitere Sensortypen zu verwenden, insbesondere Qartz-Micro-Balances- und/oder Surface-Acoustic-Wave-Sensoren.

Die Sensoren 16 sind mit einer elektrischen Beschaltung 18 verbunden, die die Sensorantworten aufbereiten und als Meßwerte dann an einen Rechner 20 weitergeben. Der Rechner 20 umfaßt einen Pool oder eine Datenbank von Fingerabdrücken des zu identifizierenden Geruchs, hier der Geruch von Fruchtwasser, sowie von Fingerabdrücken von Negativproben, hier von fruchtwasserfreiem Sekret. Unter dessen Verwendung wird z. B. unter Einsatz mathematischer Methoden eine Clusterung vorgenommen. Dazu werden in einem ersten Schritt mit Hilfe der multivariaten Datenanalyse aus den aufgenommen Meßwerten Merkmale extrahiert, die dann in einem zweiten Schritt unter Berücksichtigung einer bekannten klinischen Diagnose entsprechend ihrer Zugehörigkeit zu Gruppen (Clustern) zusammengefaßt werden. Für den zweiten Schritt werden in der Regel ebenfalls multivariate Techniken eingesetzt, wie z. B. die Hauptkomponenten-Analyse (principal compound analysis oder PCA).

Zur Identifizierung selbst werden die Sensorantworten der flüchtigen Bestandteile 8 der zu identifizierneden Flüssigkeit einem Cluster bzw. einem Geruchstyp zugeordnet, wobei im Prinzip dieselben Verfahren werden wie bei Aufbau des Datenpools. Entsprechend der Zuordnung ist damit eine Aussage möglich, ob Fruchtwasser im Vaginalsekret enthalten ist oder nicht.

Bei einer ausreichenden Empfindlichkeit der Sensoren 16 gegenüber den Fruchwasser charakterisierenden flüchtigen Biomolekülen auch bei Verdünnung durch die umgebenden Raumluft können die flüchtigen Bestandteile direkt am Patienten ohne vorherige Probennahme gemessen werden. Dazu wird, wie in FIG 2 gezeigt, die Probennahmeeinheit 10 direkt mit dem Patienten oder hier der Patientin in Verbindung gebracht.

Die Probennahmeeinheit 10 mit den Sensoren 16 einschließlich deren elektrische Beschaltung 18 und dem Auswerterechner 20 sind unter der Bezeichnung MOSES II von der Firma Lennartz in Tübingen erhältlich.

## Patentansprüche

1. Verfahren zum Identifizieren einer von einem Lebewesen abgesonderten Flüssigkeit, insbesondere von Fruchtwasser,
**dadurch gekennzeichnet,**
daß die Flüssigkeit über deren charakteristische flüchtige Bestandteile (8) identifiziert wird.

2. Verfahren nach Anspruch 1,
**dadurch gekennzeichnet,**
daß die charakteristischen flüchtigen Bestandteile (8) mehreren analytunspezifischen Sensoren (16) zugeführt werden, die untereinander verschiedenes Sensorverhalten besitzen, und daß von den flüchtigen Bestandteilen erzeugte Meßsignale der Sensoren (16) einem Rechner (20) zur Typisierung und Clusterung zugeführt werden.

3. Verfahren nach Anspruch 2,
**dadurch gekennzeichnet,**
daß die charakteristischen flüchtigen Bestandteile (8) mindestens einem Metalloxid-Sensor zugeführt werden.

4. Verfahren nach Anspruch 2 oder 3,
**dadurch gekennzeichnet,**
daß die charakteristischen flüchtigen Bestandteile (8) mindestens einem Leitfähigkeitssensor zugeführt werden.

5. Verfahren nach einem der Ansprüche 1 bis 4,
**dadurch gekennzeichnet,**
daß die charakteristischen flüchtigen Bestandteile mindestens einem Quartz-Micro-Balances-Sensor zugeführt werden.

6. Verfahren nach einem der Ansprüche 1 bis 5,
**dadurch gekennzeichnet,**
daß die charakteristischen flüchtigen Bestandteile (8) mindestens einem Surface-Acoustic-Wave-Sensor zugeführt werden.

7. Verfahren nach einem der Ansprüche 1 bis 6,
**dadurch gekennzeichnet,**
daß eine Probe der Flüssigkeit mittels eines Probenträgers (2) entnommen und einem mit den Sensoren (16) verbundenen Probenbehälter (4) zugeführt wird.

8. Verfahren nach einem der Ansprüche 1 bis 6,
**dadurch gekennzeichnet,**
daß die charakteristischen flüchtigen Bestandteile (8) direkt vom Lebewesen abgesaugt werden.

9. Vorrichtung zur Durchführung des Verfahrens nach einem der Ansprüche 1 bis 8 mit mehreren analytunspezifischen Sensoren (16), mit den Sensoren (16) verbundene Mittel (10) zur Zuführung von charakteristischen flüchtigen Bestandteilen (8) einer zu identifizierenden Flüssigkeit eines Lebewesens und einer mit den Sensoren (16) verbundenen Auswerteeinheit (20) zur Typisierung und Clusterung von den Sensoren (16) abgegebener Meßsignale.
